# EUROPEAN PATENT SPECIFICATION

(11) **EP 4 066 747 B1**
(45) Date of publication and mention of the grant of the patent: **17.07.2024**
(21) Application number: 21166346.3
(22) Date of filing: 31.03.2021
(51) Int. Cl.: A61B 8/08, A61B 8/00, G06T 7/00, G16H 50/20

(54) **METHOD AND SYSTEM FOR DETECTING OBJECTS IN ULTRASOUND IMAGES OF BODY TISSUE**
VERFAHREN UND SYSTEM ZUR DETEKTION VON OBJEKTEN IN ULTRASCHALLBILDERN VON KÖRPERGEWEBE
PROCÉDÉ ET SYSTÈME POUR DÉTECTER DES OBJETS DANS DES IMAGES À ULTRASONS D'UN TISSU CORPOREL

(43) Date of publication of application: 05.10.2022
(73) Proprietor: AURA Health Technologies GmbH, 10707 Berlin (DE)
(72) Inventor: Rieger, Jan, 04103 Leipzig (DE)
(74) Representative: Eisenführ Speiser

(56) References cited:
- US-A1- 2019 053 790
- US-A1- 2020 074 631

## Description

The invention relates to an ultrasound system that can be used for detection of an object in tissue. The invention further relates to a system that comprises a device that is configured to localize the implantable object. The invention also relates to a method and algorithm of detecting an object in ultrasound data. The invention also relates to a method of aligning a detector probe with the position of an implantable object and of navigating the user towards the implantable object.

The present invention relates generally to medical imaging, radiology and surgery and more specifically to implantable markers, clips, tags, transponders and to systems and methods for localizing such makers, tags and transponders within a patient's body e.g. during breast cancer diagnosis and therapy or e.g. during laparoscopy or in diagnostic and therapeutic procedures in general.

Medical imaging methods such as mammography, tomosynthesis or ultrasound imaging are used to identify and/or confirm the location of a lesion before a biopsy or a surgical procedure (e.g. lumpectomy).

After a biopsy procedure a biopsy marker (i.e. a small implantable object) might be placed in the location of biopsy as a reference for future follow-up examinations. If the lesion is diagnosed as benign, the biopsy marker may remain implanted in the tissue or it may resorb after certain time. If the lesion is diagnosed as malignant, the biopsy marker may be removed during the following surgery.

Prior to a surgery, to remove the malignant lesion, an additionally marking might be required. A localization wire may be placed to mark the lesion, such that a tip of the wire is positioned in a defined relationship to the lesion (e.g. either in the center of it or at the border). Once the wire is placed, the patient is transferred to operating room to perform a surgery. The surgeon then uses the wire as a guidance to access and to remove the lesion.

A know disadvantage of wire localization is the dislocation of the wire between the time of placement and the time of surgical procedure. As a consequence, the surgeon may misinterpret the location of the lesion. Additionally, in some cases, the surgeon would prefer a different access path to the lesion than the localization wire dictates.

As an alternative to localization wire, it has been suggested to mark the lesion with a radioactive seed. The radioactive seed is introduced by a needle into the lesion, the needle is withdrawn and the position of the seed is confirmed using mammography or ultrasound imaging. The surgeon then uses a radiosensitive handheld gamma probe to approximate the position of the radioactive seed. An initial incision may be made and the probe is then used to guide the surgery towards the seed and to remove the lesion with the radioactive seed inside.

Migration of the seed might as well happen between the time of placement and the surgical procedure. Thus, similarly to using a localization wire, the seed may not accurately identify the location of the lesion. Furthermore, regulatory requirements for handling radioactive seeds are an additional hurdle for employing this technique.

Alternatively, it has been proposed that other types of seeds such as magnetic, RF or RFID might be used to tag the lesion. The tags are delivered with a needle into the lesion. The surgeon then uses a compatible detector probe (magneto-detector, RF antenna etc.) to approximate the position and distance to the seed and to access and remove the lesion with it.

Similar to radioactive seeds these seeds might migrate and not precisely identify the location of the lesion. Additionally, as electromagnetic fields are not homogeneous and not isotropic, it has been reported, that some of the seed's response is directionally dependent and thus they don't provide accurate navigation from all angles.

One common disadvantage of the prior art techniques mentioned above is that the surgeon does not have any overview about the anatomy in the operating area around the marker. It has been suggested that an image guided surgery would lead to a higher precision, lower amount of resected tissue while also lowering the breast cancer recurrence. It is an object of the invention to provide an improved ultrasound system and/or an improved method for processing ultrasound data to achieve this.

According to the invention, the object is achieved with an ultrasound system that comprises a frontend for ultrasound data acquisition applying acquisition parameter values for acquisition parameters that include at least a focal depth. The ultrasound system further comprises a backend for processing ultrasound data acquired by the frontend and an image processing and display unit for image data processing and displaying,

The ultrasound system further comprises an object detector comprising a classifier that is configured to detect data representing an object in an input data set generated by the frontend and fed to the object detector.

Preferably, the object detection data generated by the object detector is fed back to the frontend for adapting acquisition parameter values depending on the data generated by the object detector.

Preferably, the classifier comprises at least one neural network that is trained with training data sets comprising data representing an object to be detected. In a more preferred embodiment, the neural network is trained to data representing an implanted marker and is trained with training data sets that comprise data representing an implanted marker. According to a further aspect, the input data set fed to the object detector comprises ultrasound data generated by the frontend and acquisition parameter values that were applied for acquiring the ultrasound data.

The ultrasound system further comprises signaling means that are operatively connected to the object detector and that are configured to emit a user perceivable signal that changes depending on an output of the object detector, the user perceivable signal being an audible sound signal and/or vibrations that can be sensed by the user.

According to a further aspect, a method of detecting an object in ultrasound data is provided. The method comprises the steps of:
- acquiring ultrasound data,
- generating ultrasound data sets comprising analog-to-digital converted ultrasound data,
- analyzing the ultrasound data sets by means of a neural network that is trained with training data sets containing ultrasound data comprising an object, in particular a marker, to be detected, and that is adapted to generate a prediction representing a likelihood that the object is represented in the analyzed ultrasound data set,
- generating a feedback signal that depends on the prediction, and
- using the feedback signal for further ultrasound data acquisition.

The method further comprises the step of generating a user perceivable signal that depends on the prediction, the user perceivable signal being an audible sound signal and/or vibrations that can be sensed by the user.

The invention includes the recognition that currently, if the surgeon wants to have image guidance for the surgery he has to alternate between an ultrasound probe for imaging and a second detection device for marker detection, e.g. a radar-probe or a magnetometer or an RFID reader. It would be useful if the object detection and imaging were combined in one improved ultrasound imaging device to facilitate image guided surgery. The combination of anatomical imaging and the marker detection into one device is one of the possible embodiments of the present invention.

### Object to be detected

The object that has to be detected preferably is an implantable marker (i.e. a biopsy marker, biopsy clip, preoperative seed, surgical target).

The system is designed to identify one particular object or different objects. Accordingly, the classifier of the object detector can be trained as a binary classifier for one object or a multi-class classifier. If more objects can be identified by the system then the objects can also be classified by the system.

The object or at least a part of it may be hyperechoic compared to its environment. As such, the object would reflect more ultrasound energy than other structures around it.

The object or at least a part of it may also be hypoechoic compared to its environment. As such, the object would reflect less ultrasound energy than other structure around it.

The object or at least a part of it may further have a distinguishable shape such as cubic, spherical, hexagonal etc. that make the object appear distinguishably in ultrasound data

The object or at least a part of it may further generate a distinguishable echo pattern that is for example achieved by layering echogenic and non-echogenic materials.

The object or at least a part of it may further generate a distinguishable echo frequency response. The distinguishable echo frequency response is for example achieved by resonant structures, i.e. magnesium blocks, that resonate at frequencies related to their dimensions. The object may comprise an ultrasound resonator or the object itself is a resonator.

The object may enclose small elements that for instance are filled into a respective cavity of the enclosure. The small elements can be ultrasound microbubbles, microshells, polymer grains, glass bubbles, gas-filled cavities within a porous material or the like. Preferably, the small elements are configured to amplify, modulate and/or filter the ultrasound waves that reach the object from the detector or the small elements may amplify, modulate and/or filter the ultrasound waves generated by the ultrasound emitter.

The object may comprise fixation means that are configured to prevent dislocation of the object. Preferably the fixation means are part of the enclosure or external to it and provide fixation by anchoring the object mechanically in soft tissue.

The object may be made of biocompatible material or coated with biocompatible material.

The object may be configured for permanent implantation, for instance in breast tissue or lymph nodes.

An advantage of an object as defined above is that the object can be used in combination with a prior art hand-held ultrasound probe.

Preferably, the object has a cross-section between 0.5 mm and 10 mm and preferably a length between 2 mm and 30 mm.

The object preferably is visible in ultrasound imaging and/or in x-ray imaging, and/or in MRI imaging.

The object preferably is an implantable object that is configured to exhibit an exclusive feature that can only be detected by the ultrasound system. For instance the object may have an unique feature the object detector of the ultrasound system is trained for.

### Ultrasound based detector

The object of the invention is achieved by an ultrasound based detector, that supports both biomedical ultrasound imaging and the detection of an object such as a biopsy marker.

The ultrasound based detector is an ultrasound system. The ultrasound based detector comprises a handheld probe, a signal processor, which comprises a frontend and a backend, a display and different elements of a user interface.

The handheld probe comprises a plurality of ultrasound transceivers and is configured to emit and receive ultrasound.

The ultrasound system comprises a frontend and a backend and is configured to generate and control ultrasound transmission and to process ultrasound signals received by the probe. "Processing ultrasound signals" means that the signal processor processes electric signals provided by the ultrasound transceivers of the handheld probe. Part of the signal processor is an object detector according to the invention.

The display is configured to show the results produced by the signal processor such as the biomedical ultrasound image and the position of the detected implanted object. The position of the detected implanted object may be detected by the object detector that may comprise a segmenting neural network for object detection and generating image data that can be overlayed to the biomedical ultrasound image. The display may also show control elements for controlling the signal processor.

Different elements of the user interface can be configured to generate different visual or other guidance perceptible by the user. The user perceivable signals providing guidance are generated in dependence of processed ultrasound signals, for instance in dependence of the prediction provided by the classifier of the object detector.

### Ultrasound probe

The ultrasound probe can be any prior art ultrasound probe comprising a linear, a sector and/or a phased array prior art ultrasound transceiver. The ultrasound probe might have a wire to connect it to the signal processor or it might be wireless and transmit the data via electromagnetic field (WiFi, Bluetooth). The probe itself has an ergonomic handle and a probe head, which houses a plurality of elements that can transmit and receive ultrasound waves. The probe head can also encompass transmitters and receivers of electromagnetic waves such as light, infrared light, radiowaves etc.

### Signal processor

The signal processor may comprise multiple units. It may comprise an ultrasound data acquisition unit, an ultrasound data processing unit, an image processing unit, a display and an object detector.

For ultrasound imaging purposes, the acquired data is processed in a way known in the art. Preferably, received electric signals from the ultrasound probe are beamformed, amplified and pre-filtered by the data acquisition unit.

Afterwards, the signals are Hilbert-transformed to calculate the quadrature component of the signals and the envelope is then calculated from the in-phase (I) and the quadrature (Q) components. The signals may then by logarithmically compressed, down-sampled, and filtered by the data processing unit. Finally further post-processing such as speckle reduction and image enhancement is performed by the image processing unit before the image is shown on the display.

### Object detection

The objective of the object detection is to find the position of an object in the data. The object detector is configured to detect and locate either one particular object or to detect and locate a plurality of objects and to distinguish the objects from each other.

The object detector can take as input the data from any of the aforementioned units. It can work with data from any stage of the data acquisition unit (frontend of the ultrasound system) or it can work with data from any stage of the data processing unit (backend of the ultrasound system) or it can work with data from any stage of the image processing and display unit of the ultrasound system or it can work with any combination of the data from any of the units.

Preferably, the input data set for the object detector comprises post-receiver-beamformer data or data after quadrature demodulation or with enveloped signals without further post-processing. The object detector can take as an input one set of data acquired using acquisitions parameter values as defined in one acquisition parameter value set. It is one aspect of the invention, that the object detector can also take as an input several sets of data that were acquired with different acquisition value sets. For example one input can be an enveloped data set acquired at one frequency with one focal depth and a second input can be a filtered data set acquired at another frequency with another focal depth.

The general process of the object detection comprises several steps. There is pre-processing of the input raw data, there is classification and there is the post-processing of the results of the classification. The results are output to the display and can also control the frontend. The steps of the object detection process are performed by corresponding units of the object detector.

The objective of the preprocessing step is to prepare the data for the classification. The objective of preprocessing might also be to decrease the number of the data by eliminating non-useful or uninteresting data by using a priori knowledge. The preprocessing for example includes steps to identify one or more regions of interest, e.g. by segmentation. These regions of interest are then passed to the classification block in the form of patches for further evaluation.

The preprocessing step may include: filtering, resampling, reshaping, quantization, feature extraction, hypothesis generation, segmentation, Fourier transformation or any other transformation of data into different representation or space. The preprocessing can be performed by defined, non-self-learning algorithms or it can be performed by machine learning models that have been previously trained or it can be performed by self-learning algorithms.

Preferably, the result from the preprocessing step is fed back to the control unit of the front-end and can influence at least one of the acquisition parameter values of the ultrasound system. For example, the result form the preprocessing step can be used to control the focal depth depending on the position of the identified regions of interest. If the regions of interest are outside of the focal depth that was used to collect the data, the next data acquisition that will be used for object classification can be initiated with the focal depth as indicated by the result from the preprocessing step. It is one aspect of a preferred embodiment, that the object detection algorithm can control the data acquisition in a feedback loop.

The objective of the classification step is to take the data after preprocessing and to classify the data, whether or not they represent or contain the object of interest. The system can be configured to distinguish a plurality of objects and classify them. In one embodiment the classification unit is additionally configured to classify abnormalities in the tissue.

In a preferred embodiment the preprocessing step or the classification steps of the object detection process are performed by one or multiple pre-trained or self-learning machine learning models that process, interpret, segment or classify data. Accordingly, in a preferred embodiment, the object detector comprises one or more neural networks that are configured and trained to implement machine learning models that can process, interpret, segment or classify data.

The machine learning models may be implemented by a feedforward multilayer perceptron neural network trained by means of a sequential backpropagation algorithm using training data sets comprising the object of interest. The object detector may implement a kernel-based method to map feature vectors into higher-dimensional space and training an optimal hyper plane to fit data.

The machine learning model could be implemented by a convolutional neural network (CNN) such as U-net that have a downsampling (encoder) and upsampling (decoder) path.

The machine learning model could be implemented by a support vector machine.

The machine learning model could implement a Viola-Jones algorithm or a type of a cascade of weak classifiers.

The machine learning model could also be implemented by an adversarial neural network, in particular a generative adversial neural network (GAN).

The machine learning models are trained with appropriate learning algorithms that may or might not include backpropagation.

In particular the classifier of the object detector may combine different classifiers, in particular a combination of classifiers trained with time domain data and classifiers trained with frequency domain data. In particular, the object detection classifier may comprise a neural network that is trained for different object classes (multi-class-classifier). In addition or alternatively, the object detector may comprise one or more neural networks that are trained as binary classifiers.

The machine learning models may be pre-trained by supervised or unsupervised learning methods or they may have self learning mechanisms and might adapt continuously.

The machine learning model may comprise multiple components, where each component can itself be considered as a standalone object detection module as defined above.

The results of the classification step are processed in the post-processing step. Post-processing may include filtering, resampling, reshaping, quantization, classification, and/or inverse Fourier transformation or any other inverse transformation of data into time domain.

### Display and elements of user interface

The display and the user interface preferably comprise multiple components for
- Controlling the signal processor, especially the data acquisition and the data processing,
- Visualization of the biomedical ultrasound image and the controls of the visualization and
- Providing user perceivable feedback from the object detector and its controls.

### Controlling the signal processor

The user interface provides all means to control the data acquisition, the data processing and the image processing. This can for example include means to set values for the acquisitions parameters, for example the transmit power, the transmit frequency, the focal depth, the time-gain-compensation levels, the depth and width of the acquired data and the receiver gain. Preferably, via the user interface, further parameter values can be set, e.g. the display bandwidth, the image enhancement methods, speckle reduction method and further image post-processing methods.

### Visualization of the biomedical ultrasound image

The visualization of the biomedical ultrasound image on the display can be any visualization known in the art of ultrasound imaging.

### Providing user perceivable feedback

The user perceivable feedback generated in dependence from the object detector output can be an audible sound signal. Additionally or alternatively a user guiding signal generator can be configured to generate vibrations that can be sensed by the user.

### Visual feedback

The visual feedback generated in dependence from the object detector output can be achieved by displaying the object position overlayed to the biomedical ultrasound image. The visual feedback generated in dependence from the object detector output can include displaying just one single coordinate (e.g. a cross-hair) or a group of coordinates (e.g. a segment).

The visual feedback generated in dependence from the object detector output can be a probability map from Bayesian probability neural network or a part of it.

The visual feedback generated in dependence from the object detector output can be achieved by displaying the object coordinates or depth independent of the biomedical ultrasound image.

### Acoustic feedback

A further aspect of the invention is providing an acoustic feedback signal generated in dependence from the object detector output in such a way that the user can work without visual feedback. The acoustic feedback may work similarly as a parking sensor in such a way that a sound generator provides an acoustic feedback signal when the probe is aligned with the implanted object and that the acoustic feedback signal varies depending on the distance between the implanted object and the probe.

One aspect of a preferred embodiment of the invention is that the ultrasound system is configured to provide acoustic feedback that depends on the size of the detected object. For a circular object, where its cross-section is maximum at the center, it would thus indicate the alignment precision between the probe and the object.

The invention shall now be further illustrated by way of exemplary embodiments with reference to the figures. Of the figures,
- Fig. 1:: is a schematic illustration of components of an ultrasound based detector system;
- Fig. 2:: is a schematic illustration of components of a signal processor according to the invention;
- Fig. 3:: illustrates a frontend of an ultrasound based detector system by way of a schematic block diagram;
- Fig. 4:: illustrates a backend of an ultrasound based detector system by way of a schematic block diagram;
- Fig. 5:: is a schematic illustration of an object detector of an ultrasound based detector system;
- Fig.: 6:illustrates an exemplary training of a neural network of the object detector;
- Fig. 7:: illustrates the exemplary structure of the neural network of the object detector; and
- Fig. 8:: illustrates an exemplary object detection by means of the neural network of the object detector.

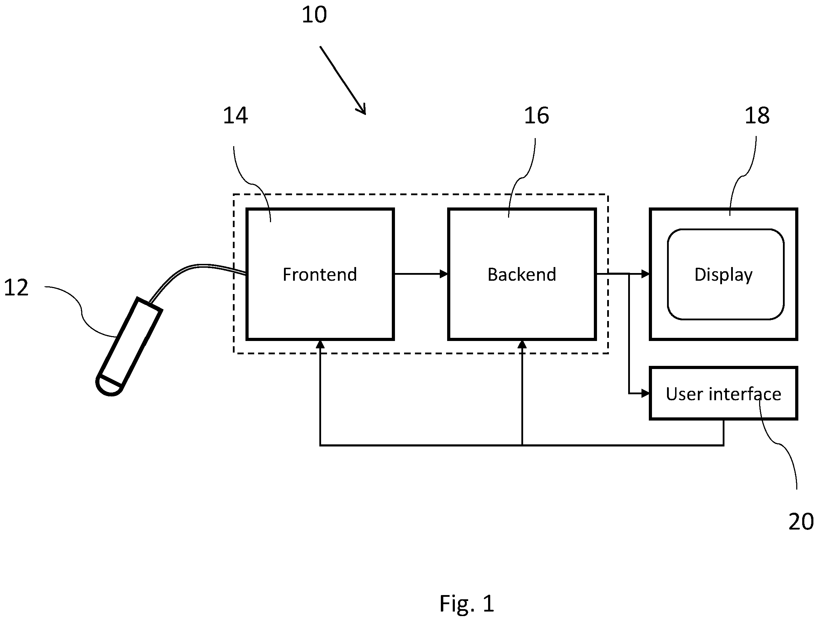

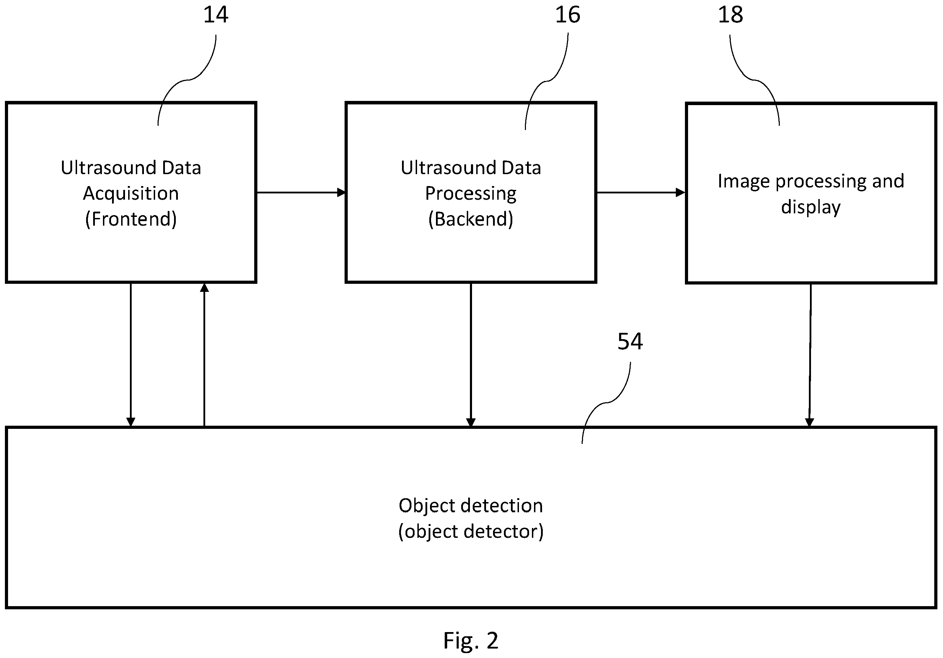

An ultrasound detector system 10 as illustrated in **figures 1** **and** 2 typically comprises a probe 12, a frontend 14, a backend 16, an image processing and display unit 18 and a user interface 20.

The probe 12 comprises a plurality of transceivers 22.

The probe 12 is connected to the frontend 14 that comprises transmitter circuits 24 for driving the plurality of transceivers 22 causing the transceivers to emit ultrasound pulses. The transmitter circuitry 24 is controlled by a control unit 26 that is configured to control the ultrasound pulses to be emitted by the transceivers 22. By way of providing a calculated phase delay between the ultrasound pulses, an ultrasound beam emitted by probe 12 can be formed and the ultrasound energy thus focused. This technique is known as beamforming. The control unit 26 causes beamforming by means of a beamforming unit 28.

The ultrasound transceivers 22 of probe 12 are further configured for receiving reflected ultrasound pulses and for converting them into electrical pulses. Accordingly, each of the plurality of transceivers 22 of probe 12 is configured to receive electrical pulses from the transmitter circuitry 26 and to put out electrical pulses that represent reflected ultrasound pulses received by a respective transceiver 22. In order to provide electrical pulses to the transceiver 22 and to receive electrical pulses from the transceivers 22 a transceiver multiplexer unit 30 is provided.

Electrical signals put out by the transceivers 22 are fed via the transceiver multiplexing unit 30 to a low noise amplifier (LNA) 32, from the low noise amplifier 32 to time-gain-compensation amplifier (TGC) 34 and from the time-gain-compensation amplifier 34 to an analogue to digital converter (ADC) 36. A plurality of analogue to digital converters 36 converts the electrical signals as provided by the transceivers 22 into digital signals that can be further processed.

Typically, processing of the digital signals comprises receiver beamforming by a digital receiver beamforming unit 38. Similar to transmitter beamforming as provided by beamforming unit 28 receiver beamforming serves for adjusting a focal depth. After receiver beamforming, the 16-bit, 32-bit or 64-bit digital signals - typically represented by a signal matrix or a signal vector - are fed to the backend 16. In the backend 16, further digital signal processing occurs. Typical steps of digital signal processing in the backend are
- quadrature modulation by a quadrature modulator 42 that applies a Hilbert-transformation to calculate the quadrature component of the signals and provides in-phase signal I and a quadrature signal Q,
- envelope detection by an envelope detector 44 that provides a positive envelope of the beamformed digital signals,
- logarithmic compression by a logarithmic compressor 46 that usually provides signals compressed to 8-bit values between 0 and 255.,
- image enhancement filtering by an image enhancement filter 48 and
- post processing by a post processor 50 that generates a display signal matrix that can be fed to image processing and display unit 18 of the ultrasound imaging system 10.

The display signal matrix generated by the scan converter 50 can be subject to further post processing.

According to the invention, an object detector 54 is provided that is connected to the frontend 16 and the backend 30 and to the image processing unit 18 of the ultrasound imaging system 10. During operation, the object detector 54 receives ultrasound data matrices from the frontend 16 or from the backend 30 or from the image processing unit 18 or from any combination of these units.

Preferably, the object detector 54 receives data from the receive-beamformer 38 or the data from envelope detection 44. Preferably, the object detector 54 further receives operating parameter values from the control unit 26 such as the delay values of the ultrasound pulses emitted by the transceivers 22.

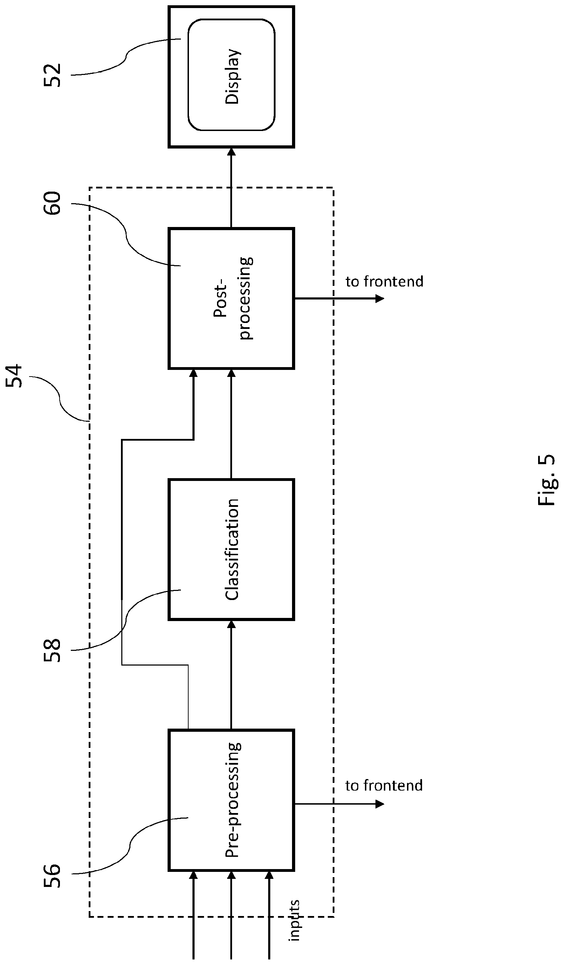

The object detection by the object detector 54 comprises several steps. There is pre-processing of the input raw data, there is classification and there is the post-processing of the results of the classification. The results are output to the display and can also control the frontend. Accordingly, the object detector 54 comprises an input data preprocessor 56, a classifier 58 and a classification result post-processor 60, c.f. **figure 5****.**

The purpose of the object detection is to find the position of the object in the data. The object detector 54 is configured to detect and locate either one particular object or to detect and locate a plurality of objects and to distinguish the objects from each other.

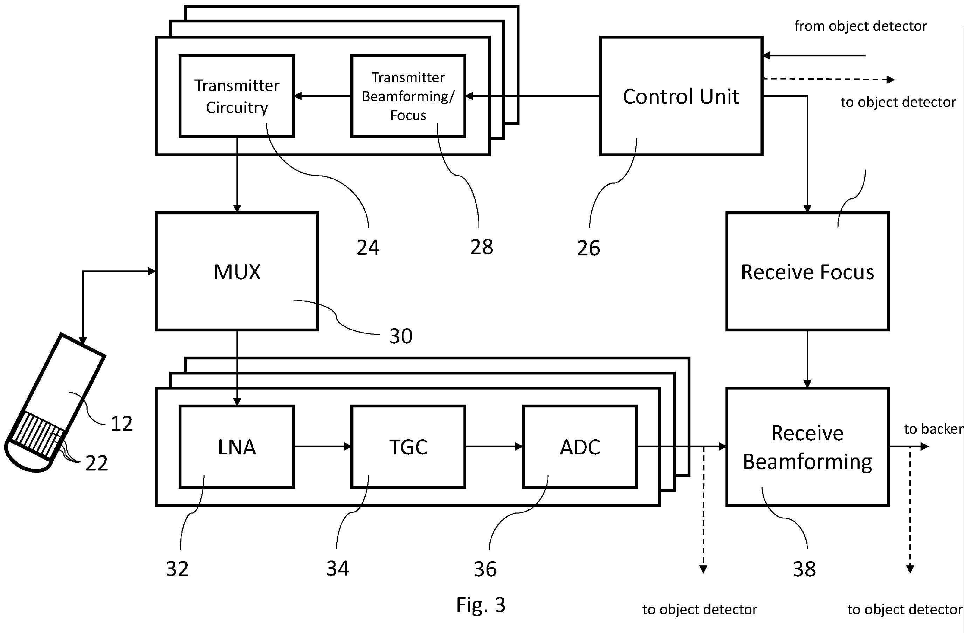

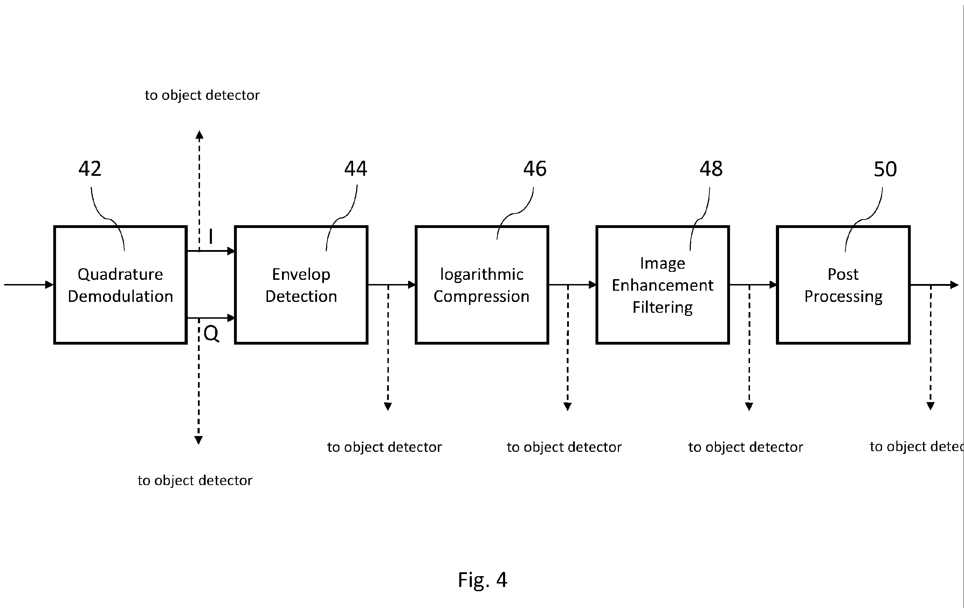

As indicated in **figures 3** **and** **4** (see: to object detector), the object detector 54 can take as input the data from any of the aforementioned units. It can work with data from any stage of the data acquisition unit (front end 14; see figure 3) or it can work with data from any stage of the data processing unit (back end 16; see figure 4) or it can work with data from any stage of the image processing unit 18 or it can work with any combination of the data from any of the units.

Preferably, an input data setforthe object detector 54 comprises post-receiver-beamformer data or data after quadrature demodulation or with enveloped signals without further post-processing. The object detector can take as an input one set of data acquired using acquisition parameter values as defined in one acquisition parameter value set. Alternatively, the object detector can processes input data sets comprising data that were acquired with different acquisition value sets. For example, one input data set can comprise data representing an enveloped data set acquired at one frequency with one focal depth and a second input data set can comprise a filtered data set acquired at another frequency with another focal depth. Frequency and focal depth are acquisition parameters. The values used for these acquisition parameters are defined in an acquisition value data set. The acquisition value data set can also be part of the input data set for the object detection. Thus, the object detector not only receives the acquired data but also the parameter values that were applied for acquisition of the acquired data.

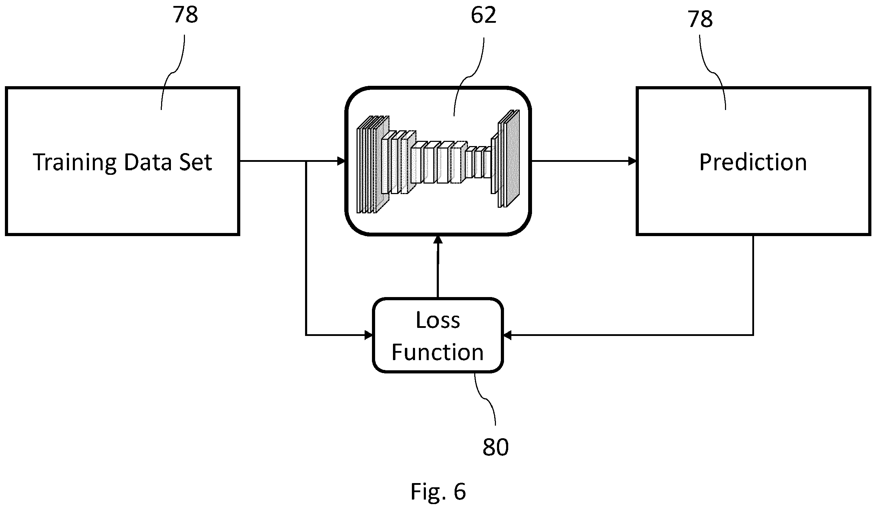

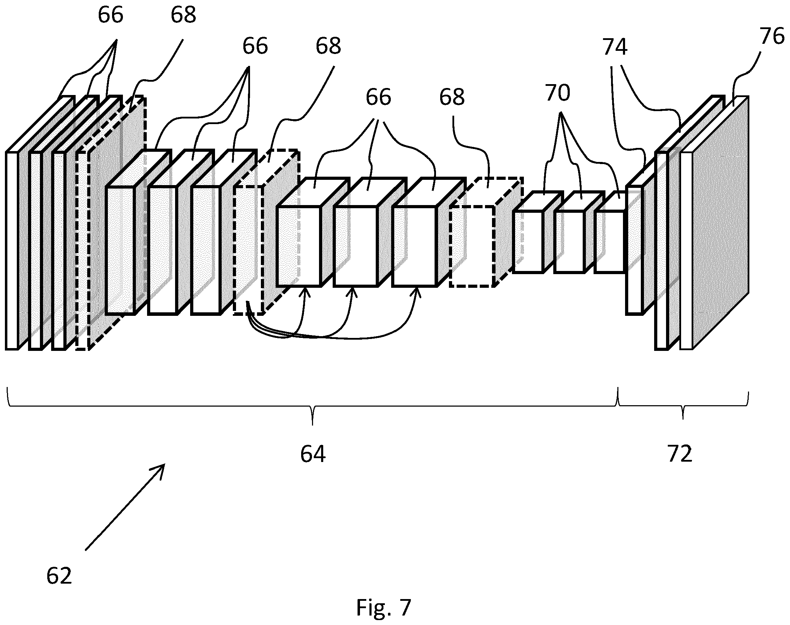

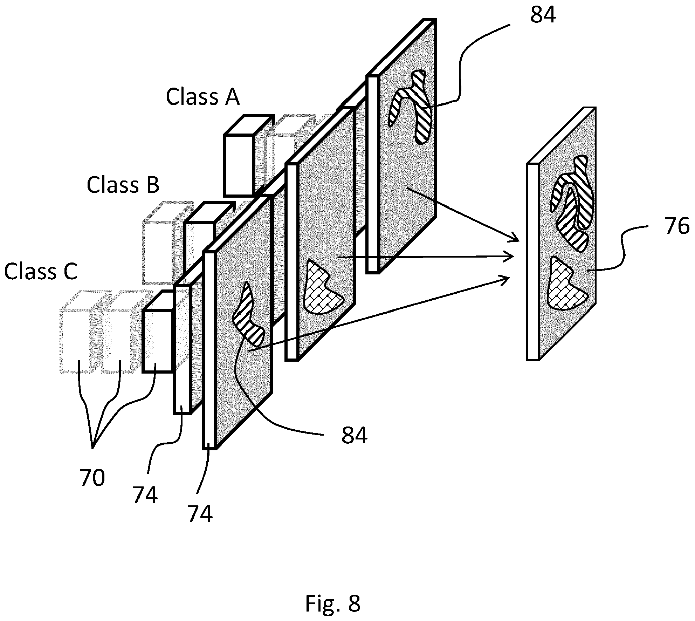

The classification is performed by the classifier of the object detector 54. The classifier preferably comprises at least one neural network as illustrated in **figures 6 to 8****.**

The results produced by the object detector 54 are output to the display 18 and can also control the frontend 14.

The purpose of the input data preprocessing unit 56 is to prepare the data for the classification. The objective of preprocessing may also be to decrease the number of the data by eliminating non-useful or uninteresting data by using a priori knowledge. The preprocessing by the input data preprocessing unit 56 for example includes steps to identify one or more regions of interest, e.g. by segmentation. These regions of interest are then passed to the classification block in the form of patches for further evaluation.

The input data preprocessing step may include: filtering, resampling, reshaping, quantization, feature extraction, hypothesis generation, segmentation, Fourier transformation or any other transformation of data into different representation or space. The preprocessing can be performed by defined, non-self-learning algorithms or by means of a machine learning input data preprocessing unit.

Preferably, the result from the preprocessing step is fed back to the control unit 26 of the front-end 14 and can influence at least one of the acquisition parameter values applied for ultrasound data acquisition For example, the results produced by the input data preprocessing unit can be used to control the focal depth depending on the position of the identified regions of interest, see "from object detector" in figure 3. If the regions of interest are outside of the focal depth the next data acquisition that will be used for object classification can be initiated with the focal depth as indicated by the result from the preprocessing step performed by the input data preprocessing unit 56. It is one aspect of a preferred embodiment, that the object detector 54 can control the data acquisition in the frontend 14 in a feedback loop.

The purpose of the classifier 58 is to take the data after preprocessing by the preprocessing unit 56 and to classify the data, whether or not they represent or contain the object of interest. The system can be configured to distinguish a plurality of objects and classify them. In one embodiment the classifier 58 is additionally configured to classify abnormalities in the tissue.

In a preferred embodiment the preprocessing step and/or the classification steps of the object detection process are performed by one or multiple pre-trained or self-learning machine learning models that process, interpret, segment or classify data. Accordingly, in a preferred embodiment, the object detector comprises one or more neural networks 62 that are configured and trained to implement machine learning models that can process, interpret, segment or classify data.

The neural network used for classification preferably is a classifier. In one embodiment also for the preprocessing step includes a neural network is provided and thus is configured for machine learning.

In one embodiment, the neural network 62 of the object detector 54 is defined by a structure of layers comprising nodes and connections between the nodes. In particular, the neural network comprises an encoder part 64 formed by convolution layers 66 and pooling layers 68. The convolutional layers 66 generate output arrays that are called feature maps 70. The elements of these feature maps 70 (i.e. arrays) represent activation levels that correspond to certain features in the ultrasound data matrix. Features generated by one layer are fed to a next convolutional layer 66 generating a further feature map corresponding to more complex features. Eventually, the activation levels of a feature map 70 correspond with the objects belonging to a class of an object the neural network was trained for.

Typically, the feature maps 70 have a smaller dimension than the input ultrasound data matrix. The encoder part 64 of the neural network thus is a classifier that can detect the presence of data representing an object (e.g. a marker) as represented by training data sets the neural network is trained with.

In the feature maps, classification scores indicate the likelihood that an object is represented in the input data set. A high classification score above a threshold thus can indicate the presence of an object while a low classification score may indicate the absence of an object. A higher the classification score indicated a better, more reliable object detection.

In order to show or highlight detected objects on a display, a decoder part 72 maybe provided. In the decoder part 72 of the neural network the feature maps are upsampled and upsampled feature maps 74 (herein also called score maps) are generated wherein the elements have score values that reflect the likelihood that a certain matrix element represents an object of the object class the neural network was trained for. The classification scores for an object can be mapped on an output matrix 76 representing an ultrasound image with highlighted detected objects - if there are any. Thus, detected objects 84, i.e. pixels having a high enough classification score for a certain object, can be highlighted.

The effect of the convolution in the convolutional layers 66 is achieved by convoluting the input array (i.e. an ultrasound data matrix as generated by the frontend and the backend of the ultrasound imaging system) with filter kernel arrays having elements that represents weights that are applied in the process of convolution. These weights are generated during training of the neural network 62 for one or more specific object classes.

Training of the neural network 62 is done by means of training data sets 78 comprising an ultrasound data matrix, optional further data, for instance parameter values of acquisition parameters of the ultrasound imaging system and labels that indicate what is represented by the ultrasound data matrix (a labeled ultrasound data matrix is called the "ground truth"). The labeled ultrasound data matrix, i.e. the ground truth, can be an ultrasound image with a highlighted detected object and thus represents the desired output, i.e. the desired prediction of the neural network.

In a back propagation process, the weights and the filter kernel arrays of the neural network 62 are iteratively adapted until the difference between the actual output of the CNN and the desired output is minimized. The difference between the actual output 82 ("prediction") of the CNN and the desired output (labeled input ultrasound data matrix of the training data set 78) is calculated by means of a loss function 80.

From a training dataset 78, containing pairs of an input ultrasound data matrix and a ground truth data set that comprises correct class labels, the neural network 62 computes the object class predictions for each element in the output matrix data set. If the neural network 62 is trained for one class (one type of object) the trained neural network 62 should be able to detect an object of that class in an ultrasound data set, for instance in an ultrasound image.

In training, the loss function 80 compares the input class labels comprised in the training data set 78 with the predictions 82 (i.e. the labels suggested by the scores in the output matrix data set) made by the neural network 62 and then pushes the parameters - i.e. the weights in the nodes of the layers - of the neural network in a direction that would have resulted in a better prediction. This is done in numerous passes (i.e. over and over again) with many different pair of input ultrasound data matrices and ground truth data sets until the neural network has learned the abstract concepts of the given object classes; cf. figure 6.

A trained neural network is thus defined by its topology of layers (i.e. the structure of the neural network), by the activation functions of the neural network's nodes and by the weights of the filter kernel arrays and potential the weights in summing up nodes of layers such as fully connected layers (fully connected layers are used in classifiers)

The topology and the activation functions of a neural network - and thus the structure of a neural network - is defined by a structure data set.

The weights that represent the specific model a neural network is trained for are stored in a model data set. The model data set must fit the structure of the neural network as defined by the structure data set. At least the model data, in particular the weights determined during training, are stored in a file that is called "checkpoint". While the structure data set is predefined by design of the neural network, the model data set is the result of training the neural network with training data sets. A model data set can be generated with any neural network having a structure as defined by the structure data set and can be transferred to another neural network having an identical structure and topology.

The model data set and the structure data set are stored in a memory that is part of or is accessible by the ultrasound imaging system.

For visualizing the prediction provided by the neural network, the image processing and display unit 18 is connected to the object detector 54.

During operation (i.e. after the training) ultrasound data matrices are fed to the object detector 54 and to the convolutional neural network 62 of the object detector 54 as input data sets. Information about the absence or presence and - if present - the position of a representation of an object in the input data set (i.e. the ultrasound data matrix) is reflected in the feature scores of the matrix elements (for instance pixels) of the predicted output matrix data set, i.e. the prediction. The feature scores can be mapped on a visual output of the ultrasound imaging system 10 to thus highlight a detected object 84 in an ultrasound image displayed on the display 52 of the image processing and display unit 18.

The trained segmenting neural network 62 has an encoder - decoder structure a schematically shown in figure 2. The encoder part 64 is a fully convolutional network (FCN).

If the neural network 62 is a mere classifier for object detection it would be implemented similarly to the encoder part 64 without an upsampling decoder part. The output, i.e. the prediction of the classifier indicates the presence or absence of data representing an object the classifier is trained for.

In case the object detector 56 detects data representing an object - for instance an implantable marker the object detector 54 was trained for - from the object detector's prediction signals can be generated that indicate either to the control unit 26 of the frontend 14 or to a user holding the probe 12 how "good" the detection is. For instance, from the object detector's prediction a signal can be generated that represents the classification score of the prediction. Such signal can be fed back to the frontend to adjust the acquisition parameter values so they lead to "better" ultrasound data sets that result in higher classification scores, i.e. a better prediction wherein the prediction represents the likelihood that the object to be detected is represented by the ultrasound data set.

Classification result postprocessing can include an analysis of the classification scores generated by the classifier 58 in combination with the acquisitions parameter values contained in the input data set fed to the object detector 54. Since acquiring ultrasound data is a dynamic process (with a plurality of ultrasound data acquisition passes) where the ultrasound probe 12 is continuously moved and acquisition parameter values may be dynamically adapted, by way of evaluating the classification result it can be found whether the detection of an object becomes better or worth over time, for instance whether the classification scores improve or decrease overtime. Accordingly, a user perceivable output signal, for instance a sound signal with varying pitch can be generated that indicates to the user the quality of the object detection. For instance, a high pitch can indicate a good object detection while a low pitch may indicate a not so good object detection. A user than can move and adapt the orientation of the ultrasound probe for improved object detection as indicated by a higher pitched sound. Thus, the probe can precisely get aligned with an implanted marker and ideal scanning parameters are chosen for making a reliable prediction about the existence of the object in the data.

From a segmented ultrasound image generated by the segmenting neural network 62, the depth of a detected object can be determined. This information can be used to generate a feedback signal for adapting the acquisitions parameter values for ultrasound data acquisition to cause the beamformer to apply a focal depth corresponding the depth the detected object has. This can lead to an improved object detection in a next ultrasound data acquisition pass.
- 10: ultrasound imaging system
- 12: probe
- 14: frontend
- 16: backend
- 18: image processing and display
- 20: user interface
- 22: transceiver
- 24: transmitter circuitry
- 26: control unit
- 28: transmitter beamforming unit
- 30: transceiver multiplexing unit
- 32: low noise amplifier
- 34: time-gain-compensation amplifier
- 36: analogue to digital converter
- 38: receiver beamforming unit
- 42: quadrature modulator
- 44: envelope detector
- 46: logarithmic compressor
- 48: image enhancement filter
- 50: post processor
- 52: display
- 54: object detector
- 56: detector input data preprocessor,
- 58: classifier
- 60: classification result post-processor
- 62: neural network
- 64: encoder part of the neural network
- 66: convolutional layer
- 68: pooling layer
- 70: feature map
- 72: decoder part of the neural network
- 74: upsampled feature map (score map)
- 76: output data matrix of the neural network
- 78: training data set
- 80: loss function
- 82: prediction
- 84: detected object

## Claims

1. Ultrasound system comprising a frontend (14) for ultrasound data acquisition applying acquisition parameter values for acquisition parameters that include at least a focal depth, a backend (16) for ultrasound data processing and an image processing and display unit (18) for image data processing and displaying,
wherein the ultrasound system comprises an object detector (54) comprising
- a classifier (58) that is configured to detect data representing an object in an input data set generated by the frontend (14) and that is fed to the object detector (54) and
- signaling means that are operatively connected to the object detector and that are configured to emit a user perceivable signal that changes depending on the output of the object detector,
**characterized in that** the user perceivable signal is an audible sound signal and/or vibrations that can be sensed by the user.

2. The ultrasound system according to claim 1, wherein the object detection data generated by the object detector (54) are fed back to the frontend (14) for adapting acquisition parameter values depending on the data generated by the object detector (54).

3. The ultrasound system according to claim 1 or 2, wherein the object detector comprises at least one neural network that is trained with training data sets comprising data representing an object to be detected.

4. The ultrasound system according to claim 3, wherein the neural network is trained to identify data representing an implanted marker and is trained with training data sets that comprise data representing an implanted marker.

5. The ultrasound system according to at least one of claims 1 to 4, wherein the input data set fed to the object detector (54) comprises ultrasound data generated by the frontend (14) and acquisition parameter values that were applied for acquiring the ultrasound data.

6. The ultrasound system according to at least one of claims 1 to 5, wherein the user perceivable signal is an audible sound signal with varying pitch that indicates to the user the quality of the object detection.

7. System comprising an ultrasound system according to at least one of claims 1 to 6 and further comprising an implantable object that is configured to exhibit an exclusive feature that can only be detected by the ultrasound system, said exclusive feature being an object's unique feature the object detector of the ultrasound system is trained for.

8. Method of detecting an object in ultrasound data, said method comprising the steps of:
- acquiring ultrasound data,
- generating ultrasound data sets comprising analog-to-digital converted ultrasound data,
- object detection by way of analyzing the ultrasound data sets by means of a neural network that is trained with training data sets containing ultrasound data comprising an object, in particular a marker, to be detected, and that is adapted to generate a prediction representing a likelihood that the object is represented in the analyzed ultrasound data set,
- generating a feedback signal that depends on the prediction,
- using the feedback signal for further ultrasound data acquisition, and
- generating a user perceivable signal that depends on the prediction wherein the user perceivable signal is an audible sound signal and/or vibrations that can be sensed by the user.

9. The method according to claim 8, wherein the user perceivable signal is an audible sound signal with varying pitch that indicates to the user the quality of the object detection.

10. The method according to claim 8 or 9, wherein the object detection comprises preprocessing of the input raw data, classification of the pre-processed input raw data and post-processing of the results of the classification.

## Patentansprüche

1. Ultraschallsystem mit einem Frontend (14) zur Ultraschalldatenerfassung, das Erfassungsparameterwerte für Erfassungsparameter anwendet, die zumindest eine Fokustiefe umfassen, einem Backend (16) zur Ultraschalldatenverarbeitung und einer Bildverarbeitungs- und -anzeigeeinheit (18) zur Bilddatenverarbeitung und -anzeige,
wobei das Ultraschallsystem einen Objektdetektor (54) aufweist, der
- einen Klassifikator (58) aufweist, der so konfiguriert ist, dass er Daten erkennt, die ein Objekt in einem vom Frontend (14) erzeugten Eingabedatensatz darstellen, und der dem Objektdetektor (54) zugeführt wird, und
- Signalisierungsmittel, die operativ mit dem Objektdetektor verbunden und so konfiguriert sind, dass sie ein vom Benutzer wahrnehmbares Signal ausgeben, das sich in Abhängigkeit von der Ausgabe des Objektdetektors ändert,
**dadurch gekennzeichnet, dass** das vom Benutzer wahrnehmbare Signal ein hörbares Tonsignal ist und/oder Vibrationen sind, die vom Benutzer wahrgenommen werden können.

2. Ultraschallsystem nach Anspruch 1, wobei die vom Objektdetektor (54) erzeugten Objekterkennungsdaten an das Frontend (14) zurückgeführt werden, um die Erfassungsparameterwerte in Abhängigkeit von den vom Objektdetektor (54) erzeugten Daten anzupassen.

3. Das Ultraschallsystem nach Anspruch 1 oder 2, wobei der Objektdetektor mindestens ein neuronales Netz aufweist, das mit Trainingsdatensätzen trainiert wird, die Daten umfassen, die ein zu erkennendes Objekt darstellen.

4. Das Ultraschallsystem nach Anspruch 3, wobei das neuronale Netz so trainiert wird, dass es Daten identifiziert, die einen implantierten Marker darstellen, und mit Trainingsdatensätzen trainiert wird, die Daten umfassen, die einen implantierten Marker darstellen.

5. Das Ultraschallsystem nach mindestens einem der Ansprüche 1 bis 4, wobei der dem Objektdetektor (54) zugeführte Eingangsdatensatz vom Frontend (14) erzeugte Ultraschalldaten und Erfassungsparameterwerte umfasst, die für die Erfassung der Ultraschalldaten angewendet wurden.

6. Das Ultraschallsystem nach mindestens einem der Ansprüche 1 bis 5, wobei das vom Benutzer wahrnehmbare Signal ein hörbares Tonsignal mit variierender Tonhöhe ist, das dem Benutzer die Qualität der Objekterkennung anzeigt.

7. System, das ein Ultraschallsystem nach mindestens einem der Ansprüche 1 bis 6 umfasst und ferner ein implantierbares Objekt umfasst, das so konfiguriert ist, dass es ein exklusives Merkmal aufweist, das nur von dem Ultraschallsystem erfasst werden kann, wobei das exklusive Merkmal ein einzigartiges Merkmal des Objekts ist, auf das der Objektdetektor des Ultraschallsystems trainiert ist.

8. Verfahren zur Erkennung eines Objekts in Ultraschalldaten, wobei das Verfahren die folgenden Schritte umfasst:
- Erfassen von Ultraschalldaten,
- Erzeugen von Ultraschalldatensätzen, die analog-digital gewandelte Ultraschalldaten umfassen,
- Objekterkennung durch Analyse der Ultraschalldatensätze mittels eines neuronalen Netzes, das mit Trainingsdatensätzen trainiert ist, die Ultraschalldaten enthalten, die ein zu erfassendes Objekt, insbesondere einen Marker, enthalten, und das geeignet ist, eine Prädiktion zu erzeugen, die eine Wahrscheinlichkeit darstellt, dass das Objekt in dem analysierten Ultraschalldatensatz repräsentiert ist,
- Generieren eines Rückkopplungssignals, das von der Prädiktion abhängt,
- Verwenden des Rückkopplungssignals für die weitere Erfassung von Ultraschalldaten, und
- Erzeugen eines vom Benutzer wahrnehmbaren Signals, das von der Prädiktion abhängt, wobei das vom Benutzer wahrnehmbare Signal ein hörbares Tonsignal ist und/oder Vibrationen sind, die vom Benutzer wahrgenommen werden können.

9. Verfahren nach Anspruch 8, wobei das vom Benutzer wahrnehmbare Signal ein hörbares Tonsignal mit variierender Tonhöhe ist, das dem Benutzer die Qualität der Objekterkennung anzeigt.

10. Verfahren nach Anspruch 8 oder 9, wobei die Objekterkennung eine Vorverarbeitung der eingegebenen Rohdaten, eine Klassifizierung der vorverarbeiteten eingegebenen Rohdaten und eine Nachverarbeitung der Ergebnisse der Klassifizierung umfasst.

## Revendications

1. Système à ultrasons comprenant une extrémité (14) frontale pour une acquisition de données d'ultrasons appliquant des valeurs de paramètre d'acquisition pour l'acquisition de paramètres, qui comprennent au moins une profondeur focale, une extrémité (16) arrière pour un traitement de données d'ultrasons et une unité (18) de traitement et d'affichage d'images pour traiter et afficher des données d'image,
dans lequel le système à ultrasons comprend un détecteur (54) d'objet, comprenant
- un classificateur (58), qui est configuré pour détecter des données représentant un objet en un ensemble de données d'entrée créé par l'extrémité (14) frontale et qui est envoyé au détecteur (54) d'objet et
- des moyens de signalisation, qui sont reliés fonctionnellement au détecteur d'objet et qui sont configurés pour émettre un signal perceptible par un utilisateur, qui change en fonction de la sortie du détecteur d'objet,
**caractérisé en ce que** le signal perceptible par un utilisateur est un signal sonore audible et/ou des vibrations, qui peuvent être détectées par l'utilisateur.

2. Système à ultrasons suivant la revendication 1, dans lequel les données de détection d'objet, créées par le détecteur (54) d'objet, sont renvoyées à l'extrémité (14) frontale pour adapter des valeurs de paramètre d'acquisition, en fonction des données créées par le détecteur (54) d'objet.

3. Système à ultrasons suivant la revendication 1 ou 2, dans lequel le détecteur d'objet comprend au moins un réseau neuronal, qui subit un apprentissage par des ensembles de données d'apprentissage comprenant des données représentant un objet à détecter.

4. Système à ultrasons suivant la revendication 3, dans lequel le réseau neuronal subit un apprentissage pour identifier des données représentant un repère implanté et subit un apprentissage par des ensembles de données d'apprentissage, qui comprennent des données représentant un repère implanté.

5. Système à ultrasons suivant au moins l'une des revendications 1 à 4, dans lequel l'ensemble de données d'entrée, envoyé au détecteur (54) d'objet, comprend des données d'ultrasons créées par l'extrémité (14) frontale et des valeurs de paramètre d'acquisition, qui sont appliquées pour acquérir les données d'ultrasons.

6. Système à ultrasons suivant au moins l'une des revendications 1 à 5, dans lequel le signal perceptible par un utilisateur est un signal sonore audible de hauteur variable, qui indique à l'utilisateur la qualité de la détection de l'objet.

7. Système à ultrasons suivant au moins l'une des revendications 1 à 6, et comprenant en outre un objet implantable, qui est configuré pour présenter une particularité exclusive, qui peut être détecté seulement par le système à ultrasons, ladite particularité exclusive étant une particularité unique d'objet, pour laquelle le détecteur d'objet du système à ultrasons subit un apprentissage.

8. Procédé de détection d'un objet dans des données d'ultrasons, le procédé comprenant les stades de :
- acquisition de données d'ultrasons,
- création d'ensembles de données d'ultrasons comprenant des données d'ultrasons transformées d'analogique à numérique,
- détection d'objet par le biais d'une analyse d'ensembles de données d'ultrasons au moyen d'un réseau neuronal, qui subit un apprentissage par des ensembles de données d'apprentissage contenant des données d'ultrasons comprenant un objet, en particulier un repère, à détecter, et qui est agencé pour créer une prévision représentant une probabilité que l'objet soit représenté dans l'ensemble de données d'ultrasons analysé,
- création d'un signal de réaction, qui dépend de la prévision,
- utilisation du signal de réaction pour une autre acquisition de données d'ultrasons, et
- création d'un signal perceptible par un utilisateur, qui dépend de la prévision, dans lequel le signal perceptible par un utilisateur est un signal sonore audible et/ou des vibrations, qui peuvent être détectées par l'utilisateur.

9. Procédé suivant la revendication 8, dans lequel le signal perceptible par un utilisateur est un signal sonore audible de hauteur variable, qui indique à l'utilisateur la qualité de la détection de l'objet.

10. Procédé suivant la revendication 8 ou 9, dans lequel la détection d'objet comprend un pré-traitement des données brutes d'entrée, une classification des données brutes d'entrée pré-traitées et un post-traitement des résultats de la classification.
